# EUROPÄISCHE PATENTSCHRIFT

(11) **EP 1 232 203 B1**
(45) Veröffentlichungstag und Bekanntmachung des Hinweises auf die Patenterteilung: **21.07.2004**
(21) Anmeldenummer: 00967842.6
(22) Anmeldetag: 06.10.2000
(51) Int. Cl.: C08J 9/28, A61L 27/20, C12N 5/00

(54) **3D-MATRIX ZUR HERSTELLUNG VON ZELLTRANSPLANTATEN**
3D MATRIX FOR PRODUCING CELL TRANSPLANTS
MATRICE EN 3D POUR PREPARER DES TRANSPLANTS CELLULAIRES

(30) Priorität: 06.10.1999 DE 19948120
(43) Veröffentlichungstag der Anmeldung: 21.08.2002
(73) Patentinhaber: Alvito Biotechnolgie GmbH, 14943 Luckenwalde (DE)
(72) Erfinder: HAHNEMANN, Birger, 15806 Zossen (DE); PAHMEIER, Andrea, 15806 Zossen (DE); LÖTZBEYER, Thomas, 27574 Bremerhaven (DE)
(74) Vertreter: Hoffmann, Jörg Peter, Dr. Ing.
(86) Internationale Anmeldenummer: PCT/EP2000/009809
(87) Internationale Veröffentlichungsnummer: WO 2001/025321

(56) Entgegenhaltungen:
- EP-A- 1 053 739
- DATABASE WPI Week 199515 Derwent Publications Ltd., London, GB; AN 1995-110731 XP002157156 "Making chitosan porous matter used e.g. for sepn. of mixed solns. - comprising cooling and freezing acidic aq. soln. of chitosan and drying by subliming water without thawing." & JP 07 033902 A (LIGNYTE CO LTD), 3. Februar 1995 (1995-02-03)
- DATABASE WPI Week 199049 Derwent Publications Ltd., London, GB; AN 1990-364546 XP002157157 "Prodn. of porous chitosan material for filters, etc. - by dissolving chitosan in aq. acid soln., freeze drying, neutralising etc." & JP 02 261838 A (SUMITOMO CEMENT CO.), 24. Oktober 1990 (1990-10-24)
- DATABASE WPI Week 199039 Derwent Publications Ltd., London, GB; AN 1990-294670 XP002157158 "Porous carrier for cell culture - comprises high molecular substance having membrane partitioned micropores" & JP 02 207785 A (ASAHI CHEM IND CO LTD), 17. August 1990 (1990-08-17)

## Beschreibung

Die Erfindung betrifft ein Verfahren zur Herstellung einer biologisch verträglichen dreidimensionalen Matrix, eine mit dem erfindungsgemäßen Verfahren erhältliche Matrix, sowie deren Verwendung.

Auf dem Gebiet der Transplantationsmedizin sind in den letzten Jahren erhebliche Erfolge erzielt worden. Probleme bereiten jedoch die geringen verfügbaren Mengen an Spenderorganen, was zu erheblichen Wartezeiten für transplantationspflichtige Patienten geführt hat. Ein weiteres Problem besteht darin, dass mit den Spenderorganen auch Krankheitserreger übertragen werden können, wie dies zumindest zeitweise bei Spendern der Fall war, die an AIDS erkrankt waren. Ferner werden transplantierte Organe vom Immunsystem des Empfängers als fremd erkannt. Nach erfolgter Transplantation ist der Patient daher auf immunsuppressive Medikamente zur Unterdrückung von Abstoßungsreaktionen angewiesen. In jüngerer Zeit sind Versuche unternommen worden, künstliche Organe aus Zellkulturen zu züchten, wobei in einigen Bereichen beträchtliche Erfolge erzielt werden konnten. Dabei werden Zellen auf einer dreidimensionalen Matrix kultiviert, die entsprechend den Bedürfnissen geformt werden kann, beispielsweise in Form eines Ohrs. Dieses künstliche Organ oder Körperteil kann dann transplantiert werden, wobei bei Verwendung körpereigener Zellen diese nicht als fremd vom Immunsystem des Empfängers erkannt werden, also auch keine Abstoßungsreaktion eintreten kann.

Als Material für die Matrix sind beispielsweise Collagen oder Alginat im Gemisch mit Chitosan bekannt. Insbesondere Collagen wird jedoch wegen der Herstellung aus Rinderknorpel und der damit verbundenen BSE-Gefahr nur ungern für die Herstellung von Implantaten für menschliche Patienten verwendet.

Als vielversprechendes Matrixmaterial hat Chitosan ein immer größeres Interesse gefunden. Chitosan ist ein teilweise deacetyliertes Chitin und wird aus den Exoskeletten von Arthropoden gewonnen. Es ist ein Aminopolysaccharid (Poly-1-4-glucosamin), das beispielsweise im Medizinbereich als Nahtmaterial oder zur Verkapselung von Pharmaka verwendet wird. Sein Vorteil liegt darin, dass es vom Körper vollständig resorbiert werden kann.

Chitosan kann im leicht Sauren (pH < 6) durch Protonierung der freien Aminogruppen in Wasser gelöst werden. Im Alkalischen (pH > 7) fällt es aus der wässrigen Lösung wieder aus. Durch diesen pH-abhängigen Mechanismus kann Chitosan unter milden Bedingungen gereinigt und verarbeitet werden.

In der US 5.871.985 wird ein Träger für die Transplantation in einen Patienten vorgeschlagen, der aus einer Matrix besteht, in die Zellen eingewachsen sind. Dazu wird zunächst eine Lösung aus Chitosan hergestellt, in der lebende Zellen enthalten sind. Diese Lösung wird dann in eine semipermeable Membran eingeschlossen, um den Träger auszubilden. Das Chitosan wird präzipitiert und bildet eine unvernetzte Matrix aus in der die Zellen verteilt sind.

In der EP 1 053 739 A1 wird ein Verfahren zur Herstellung einer knochenbildenden Substanz beschrieben. Dabei wird zunächst ein Chitosansol bei einem sauren pH hergestellt. Zum Sol wird anschließend pulverförmiger Apatit zugegeben und die Mischung geknetet, um eine innige Durchmischung zu erreichen. Die Mischung wird anschließend neutralisiert, so dass ein pH von 7,0 bis 10 eingestellt wird. Nach Entfernen des Wassers in einem Trockenschritt wird ein Material erhalten, das unter Einwirkung äußerer Kräfte leicht zerbricht. Durch Zugabe von Ringer-Lösung kann das Material in eine elastische, gummiartige Konsistenz überführt werden.

Madihally et al. (Biomaterials 1999; 20(12), S. 1133 - 42) beschreibt eine Matrix für die Geweberegeneration. Chitosan, das zu 85 - 90 % deacetyliert ist wird dazu in 0,2 M Essigsäure gelöst, sodass Lösungen mit einem Gehalt von 1 bis 3 Gew.-% Chitosan erhalten werden. Die Lösung wird eingefroren und das Wasser und die überschüssige Essigsäure durch Lyophilisieren entfernt. Durch die Lyophilisationsbedingungen kann dabei die Form der sich bildenden Poren beeinflusst werden. Der mittlere Porendurchmesser der erhaltenen Matrix liegt im Bereich von 40 - 250 µm. Die frisch lyophilisierte Matrix wird als steif und unelastisch beschrieben. Beim Hydratisieren in einem neutralen wässrigen Medium quillt das Chitosan rasch und löst sich schließlich auf. Ein erneutes Auflösen der Chitosanstruktur kann durch Äquilibrieren in verdünnter NaOH oder durch Waschen mit einer Ethanolreihe, beispielsweise 100, 70, 50, 0 %, verhindert werden. Die lyophilisierte Matrix wird dazu für ca. 10 Minuten in 0,05 M NaOH äquilibriert und mit Wasser und PBS (phosphatgepufferte physiologische Kochsalzlösung) gewaschen. Beim Äquilibrieren in verdünnter NaOH schrumpft die Matrix jedoch und zeigt Veränderungen in ihrem Aufbau, die bei einer Erniedrigung des pH auf 7 nur zum Teil reversibel sind. Ferner zeigt die Matrix zahlreiche Lufteinschlüsse. Bessere Ergebnisse werden bei einer Äquilibrierung mit einer Ethanolreihe erhalten. Zur Entfernung von Lufteinschlüssen wird beim Waschen mit absolutem Alkohol zunächst kurz ein Vakuum angelegt. Als weiterer Vorteil wird angeführt, dass durch die Behandlung mit Alkohol die Matrix während der Äquilibrierung sterilisiert wird. Dennoch ist das Verfahren vergleichsweise aufwändig. Auch bei einer Stabilisierung der Membran durch eine Ethanolreihe findet eine Veränderung der Porenstruktur statt, da durch das Wasser das Chitosan lokal aufgelöst werden kann und damit die Porenstruktur partiell zerstört wird. Die hydratisierte Matrix wird als weich und flexibel beschrieben, zeigt jedoch nur eine geringe Festigkeit. Dies erschwert die Verarbeitung der Matrix erheblich. Wird diese beispielsweise für die Kultivierung von Knorpelzellen zur Bildung von Knorpel in die entsprechende Form zugeschnitten, zerbricht die Matrix sehr leicht. Ebenso wird die Kultivierung der Zellen erschwert, da die Matrix durch Manipulationen, beispielsweise beim Umbetten in frisches Kulturmedium, leicht in mehrere Bruchstücke zerfällt. Damit lassen sich mit bekannten Chitosanmatrices nur sehr schwer Implantate mit einer definierten Struktur herstellen. Weiter beschreiben Madihally et al. die Herstellung von Mikrocarriern. Dazu werden entweder Tropfen der wässrigen Chitosanlösung direkt beispielsweise in flüssigem Stickstoff eingefroren oder vor dem Einfrieren im Alkalischen durch NaOH als Gel ausgefällt. Größere Strukturen werden nicht beschrieben.

Der Erfindung liegt die Aufgabe zugrunde. ein Verfahren zur Herstellung einer biologisch verträglichen, dreidimensionalen Matrix zur Verfügung zu stellen, das einen geringeren Aufwand erfordert und zu stabilen, weichen Matrices führt.

Die Aufgabe wird gemäß einer ersten Ausführungsform der Erfindung gelöst mit einem Verfahren zur Herstellung einer biologisch verträglichen dreidimensionalen Matrix, wobei aus Chitosan und einem Überschuss einer Säure eine wässrige Lösung hergestellt wird, die wässrige Lösung neutralisiert wird, die neutralisierte wässrige Lösung eingefroren wird und das Wasser unter vermindertem Druck absublimiert wird.

Eine dreidimensionale Matrix mit vergleichbaren Eigenschaften wird gemäß einer zweiten Ausführungsform der Erfindung erhalten mit einem Verfahren zur Herstellung einer biologisch verträglichen dreidimensionalen Matrix, wobei aus Chitosan und einem Überschuss einer Säure, die ausgewählt ist aus der Gruppe, die gebildet ist von Alkyl- und Arylhydroxycarbonsäuren, eine wässrige Lösung hergestellt wird, die wässrige Lösung eingefroren wird und das Wasser unter vermindertem Druck absublimiert wird, wobei überschüssige Säure vor oder nach dem Absublimieren entfernt wird.

Nach dem erfindungsgemäßen Verfahren wird zunächst eine wässrige Lösung eines teilweise deacetylierten Chitosans und einer im Überschuss vorliegenden Säure hergestellt. Unter Überschuss wird dabei verstanden, dass der pH der wässrigen Lösung im Sauren liegt, vorzugsweise unterhalb von pH ≤ 4. Dadurch sind die freien Aminogruppen des Chitosans zumindest teilweise protoniert, wodurch die Löslichkeit in Wasser gesteigert wird. Die Säuremenge ist nicht kritisch. Sie muss lediglich so gewählt sein, dass das Chitosan in Lösung geht. Eine übermäßige Säurezugabe wird nach Möglichkeit vermieden, da überschüssige Säure wieder entfernt werden muss, und dadurch die Aufarbeitung bei großen Säuremengen erschwert wird. Günstig sind Säuremengen, die eine 0,05 bis 1 N, vorzugsweise 0,1 bis 0,5 N, insbesondere 0,1 bis 0.3 N Lösung ergeben.

Als Säure besonders geeignet ist Milchsäure. Die fertiggestellte Matrix enthält dann Lactationen als Gegenionen des Chitosans. Die Matrix ist in diesem Fall besonders weich und elastisch.

Die Chitosanmenge wird vorzugsweise so gewählt, dass sich eine Konzentration von 0,01 bis 5 Gewichts-%, vorzugsweise 0,5 bis 1 Gewichts-% ergibt. Durch die Konzentration der Chitosanlösung kann Einfluss auf die Struktur der Matrix, insbesondere deren Porengröße genommen werden. Auf diese Weise lässt sich die Porengröße der Matrix auf den jeweiligen Zelltypus abstimmen, mit dem die Matrix besiedelt werden soll.

Chitosan hat wegen seiner Herstellung aus natürlichen Quellen kein einheitliches Molekulargewicht. Je nach Quelle und Aufbereitungsverfahren kann das Molekulargewicht zwischen 20 kDa bis über 1.000 kDa betragen. Für die Herstellung der dreidimensionalen Matrix ist das Chitosan hinsichtlich seines Molekulargewichts keinen Beschränkungen unterworfen.

Nach dem Einfrieren wird das Wasser unter vermindertem Druck absublimiert. Geeignete Druckbereiche sind 0,001 bis 3 hPa. Die genauen Bedingungen werden von der Zusammensetzung der wässrigen Lösung beeinflusst. Sofern eine flüchtige Säure verwendet wurde, kann diese beim Lyophilisieren zumindest teilweise mit verdampft werden.

Bevorzugt wird vor dem Einfrieren zur Entfernung überschüssiger Säure durch Zugabe einer Base der pH-Wert der wässrigen Lösung auf 5,0 bis 7,5, vorzugsweise 5.5 bis 7,0, insbesondere 6,0 bis 7,0 eingestellt. Es wird eine Matrix mit sehr guten Eigenschaften hinsichtlich ihrer Formstabilität erhalten. Die Matrix schrumpft beim Rehydratisieren nicht und zeigt auch keine Strukturveränderungen. Besonders ausgeprägt ist dieses stabile Verhalten, wenn die wässrige Lösung vor dem Einfrieren einen pH von 6,0 bis 7,0 aufweist. In diesem Bereich beginnt das Chitosan auszufallen. Es wird angenommen, dass sich im Neutralen ein Gel oder eine feine Suspension ausbildet, die sich positiv auf die Struktur der lyophilisierten Matrix auswirkt. Die neutralisierte wässrige Lösung des Chitosans bildet also eine Suspension. Je nach Konzentration des Chitosans kann die wässrige Lösung beim Neutralisieren daher trüb werden.

Bei einem Verfahren, wie es von Madihally et al. vorgeschlagen wurde, erfährt die Matrix bei der Neutralisation überschüssiger Essigsäure durch Äquilibrieren mit wässriger NaOH eine starke Veränderung der Struktur. Vermutlich führt die Zugabe der wässrigen Lösung lokal zur Auflösung des Chitosans, wobei dieses durch die NaOH wieder ausgefällt wird. Damit wird die Porenstruktur der Matrix verändert.

Mit dem von den Erfindern aufgefundenen Verfahren ist nun eine Möglichkeit aufgefunden worden, die Matrix zu stabilisieren, sodass nach der Entfernung des Wassers durch Lyophilisieren keine bzw. wesentlich geringere Strukturänderungen bei der weiteren Verarbeitung der Matrix auftreten.

Bei der ersten Ausführungsform des erfindungsgemäßen Verfahrens weist die Chitosanlösung bereits vor dem Einfrieren und Lyophilisieren einen neutralen pH auf. Als "neutraler pH" ist dabei ein pH im oben angegebenen Bereich zu verstehen. Nach dem Lyophilisieren muss daher die Matrix auch nicht mehr mit Base äquilibriert werden. Wesentlich ist, dass die Chitosanlösung ausreichend neutralisiert wird und das Chitosan als Gel ausfällt. Wird die Neutralisation nicht sorgfältig ausgeführt, löst sich die erhaltene Matrix beim Rehydratisieren wieder auf. Bei der ersten Ausführungsform des erfindungsgemäßen Verfahrens ist die Säure zunächst keinen besonderen Beschränkungen unterworfen.

Für die Herstellung der wässrigen Chitosanlösung kann eine Säure verwendet werden, die ausgewählt ist aus der Gruppe, die gebildet ist von anorganischen Säuren und organischen Säuren, vorzugsweise Alkyl- und Arylcarbonsäuren, insbesondere Hydroxycarbonsäuren. Besonders bevorzugt ist Milchsäure.

Beispiele für anorganische Säuren sind Salzsäure, Phosphorsäure und saure Phosphatsalze. Beispiele für organische Säuren sind Alkylcarbonsäuren mit 1 bis 12 Kohlenstoffatomen, wobei die Alkylkette geradkettig oder verzweigt sein kann. Beispiele sind Ameisensäure, Essigsäure, Propionsäure, Buttersäure, Valeriansäure, Hexansäure, Heptansäure und höhere geradkettige Carbonsäuren. Geeignet sind auch Dicarbonsäuren mit 2 bis 8 Kohlenstoffatomen, wie Oxalsäure, Bernsteinsäure oder Adipinsäure. Geeignet sind auch aromatische Carbonsäuren, wie Benzoesäure oder Naphthoesäuren. Ferner sind auch Aminosäuren, wie Glutaminsäure oder Asparaginsäure, geeignet.

Besonders gute Ergebnisse werden jedoch mit Hydroxycarbonsäuren erhalten. Geeignet sind insbesondere Hydroxycarbonsäuren mit 2 bis 12 Kohlenstoffatomen. Es können ein oder mehrere Hydroxylgruppen sowie ein oder mehrere Carboxylgruppen im Molekül vorhanden sein. Beispiele sind Glycolsäure, Milchsäure, Äpfelsäure, Weinsäure, sowie Zitronensäure. Es können auch aromatische Hydroxycarbonsäuren verwendet werden, beispielsweise Mandelsäure.

Als Basen können übliche Basen, wie z. B. Alkalihydroxide, wie NaOH, verwendet werden. Die Basen sollten so gewählt werden, dass ohne Weiteres ein neutraler pH eingestellt werden kann.

Eine weitere Möglichkeit, eine Matrix mit verbesserter Struktur zu erhalten, besteht darin, die Matrix bereits durch die zum Auflösen des Chitosans in Wasser verwendete Säure zu stabilisieren.

Überraschend hat sich gezeigt, dass durch die Wahl des Gegenions die Eigenschaften der Chitosanmatrix beeinflusst werden können. Dies wird bei der zweiten Ausführungsform des erfindungsgemäßen Verfahrens genutzt.

Als Säuren werden dabei Hydroxycarbonsäuren verwendet. Insbesondere bei Verwendung von Milchsäure, bei dem das Lactatanion das Gegenion zur protonierten Aminogruppe des Chitosans bildet, werden sehr gute Eigenschaften erhalten. Die Matrix zeigt eine hohe Temperaturbeständigkeit und kann beispielsweise problemlos autoklaviert werden. Dabei treten keine Verfärbungen oder sonstige Zersetzungserscheinungen der Matrix auf. Die Matrix zeigt auch eine hohe Elastizität und mechanische Belastbarkeit. So lässt sich eine flächige Matrix, die durch Ausgießen der wässrigen Chitosan/Milchsäurelösung in einer Petrischale und anschließendem Einfrieren und Lyophilisieren erhalten wird, problemlos aufrollen, ohne dass die Matrix bricht.

Bei der zweiten Ausführungsform des erfindungsgemäßen Verfahrens tritt der stabilisierende Effekt der Hydroxyalkyl- bzw. -arylcarbonsäuren bereits auf, wenn die Chitosanlösung vor dem Einfrieren nicht neutralisiert wird. Nach dem Lyophilisieren werden noch vorhandene Reste überschüssiger Säure durch Äquilibrieren mit Base, z. B. 0,05 N NaOH, entfernt. Anschließend wird die Matrix noch mit Wasser, das einen Phosphatpuffer enthalten kann (0.1 N, pH ca. 7) gewaschen. Die Strukturveränderungen der Matrix fallen dabei erheblich geringer aus als bei der von Madihally beschriebenen Matrix. Besonders vorteilhaft wird bei der zweiten Ausführungsform des erfindungsgemäßen Verfahrens die Chitosanlösung bereits vor dem Einfrieren neutralisiert. Ein Äquilibrieren im Alkalischen ist dann nicht mehr erforderlich. Die Matrix kann direkt weiterverarbeitet und beispielsweise mit Zellen besiedelt werden.

Mit beiden Ausführungsformen des erfindungsgemäßen Verfahrens wird eine Matrix mit einer gleichmäßigen Porenstruktur erhalten, die weich und nachgiebig ist. Wird die Matrix beispielsweise mit Fingerdruck zusammengepresst, kehrt sie nach Aufheben des Drucks wieder elastisch in ihre ursprüngliche Form zurück. Die Matrix lässt sich verformen ohne zu brechen oder zu zerbröseln und lässt sich beispielsweise aufrollen.

Zur Ausbildung von zusammenhängenden Zellverbänden ist es erforderlich, dass die Matrix während der Besiedelung durch Zellen eine ausreichende Beständigkeit aufweist und erst über einen längeren Zeitraum abgebaut wird. Die Abbaugeschwindigkeit kann über den Deacetylierungsgrad des Chitosans gesteuert werden. Bei Verwendung eines Chitosans, das noch einen sehr hohen Acetylierungsgrad aufweist, lassen sich Abbauzeiten von mehr als einem Monat verwirklichen.

Eine besonders hohe Resorbierbarkeit der Matrix wird beobachtet, wenn das Chitosan einen Deacetylierungsgrad von ≤ 80 % aufweist. Der Deacetylierungsgrad bezieht sich auf die N-Acetylgruppen des Chitosans. Unter Deacetylierungsgrad wird das Verhältnis der nicht acetylierten Aminogruppen zu den insgesamt vorhandenen Aminogruppen verstanden (Summe aus freien Aminogruppen und acetylierten Aminogruppen). Es wird angenommen, dass bei einem Deacetylierungsgrad von ≤ 80 % in weiten Bereichen der Matrix amorphe Strukturen ausgebildet werden. Diese amorphen Bereiche können vermutlich von Enzymen besser angegriffen werden. Damit kann die Geschwindigkeit, mit der die Matrix im Körper resorbiert wird, beeinflusst und auf die jeweiligen Bedürfnisse abgestimmt werden.

Die Porengröße der Matrix kann weiter durch die Geschwindigkeit beeinflusst werden, mit der die wässrige Chitosanlösung eingefroren wird. Dabei ergibt ein rasches Einfrieren, beispielsweise in flüssigem Stickstoff kleine Porendurchmesser, während langsames Einfrieren, beispielsweise im Bereich von -30 bis -15 °C größere Poren ergibt. Bevorzugt wird die Temperatur zum Einfrieren der Chitosanlösung im Bereich von -200 °C bis -0 °C, vorzugsweise -90 °C bis -10 °C, insbesondere bevorzugt -40 °C bis -15 °C gewählt.

Die mit dem oben beschriebenen Verfahren erhältliche Matrix weist hervorragende Eigenschaften gegenüber den aus dem Stand der Technik bekannten Matrices auf. Gegenstand der Erfindung ist daher auch eine Matrix, wie sie mit dem oben beschriebenen Verfahren erhalten wird. Die Matrix ist formstabil, weich und flexibel und kann dadurch leicht verarbeitet und beispielsweise einfach in die gewünschte Form zugeschnitten werden. Sie kann autoklaviert und dadurch einfach sterilisiert werden, ohne das Zersetzungsreaktionen auftreten. Zur Stabilisierung der Matrix sind keine weiteren Substanzen, wie Collagen oder Alginat erforderlich. Der Acetylierungsgrad und damit beispielsweise die Resorbierbarkeit der Matrix ist frei wählbar und damit auf die jeweiligen Erfordernisse einstellbar. Durch die Konzentration der wässrigen Chitosanlösung und die Einfrierbedingungen kann die Struktur der Matrix, insbesondere die Porengröße beeinflusst werden.

Eine sehr formstabile und flexible Matrix wird erhalten, wenn die Matrix ein Lactat-Anion als Gegenion aufweist.

Durch die große Anzahl von Aminogruppen ist die Matrix beliebig modifizierbar. Bei einer bevorzugten Ausführungsform der dreidimensionalen Matrix sind Liganden kovalent oder nicht kovalent an die Chitosanmatrix gebunden, vorzugsweise an die freien Aminogruppen des Chitosans. Als Liganden können z. B. Wachstumsstoffe, Proteine, Hormone, Heparin, Heparansulfate, Chondroitinsulfate, Dextransulfate oder eine Mischung dieser Substanzen verwendet werden. Die Liganden dienen vorzugsweise zur Kontrolle und Verbesserung der Zellproliferation.

Das Zellwachstum auf der Matrix wird weiter verbessert, wenn die Matrix mit autologem Fibrin beschichtet ist.

Die erfindungsgemäße dreidimensionale Matrix kann als Festphase in einem Kulturreaktor (Cell Factory) verwendet werden. Die Matrix zeigt eine sehr hohe Beständigkeit im Kulturmedium. Ferner hat sich gezeigt, dass die Matrix das Zellwachstum fördert.

Die Matrix regt das Zellwachstum an. Sie kann daher direkt, d. h. ohne vorherige Besiedlung durch Zellen implantiert werden. Nach der Implantierung wachsen körpereigene Zellen ein, wobei die Matrix allmählich aufgelöst wird.

Die Matrix eignet sich ferner zur Verwendung als Zellimplantat, insbesondere für knorpelbildende Zellen. Es werden dabei bevorzugt keine gentechnisch veränderten Zellen verwendet. Die Zellen werden vorzugsweise durch Biopsie dem Patienten entnommen, auf der Zellmatrix angezüchtet und das Zellimplantat dann dem Patienten eingepflanzt. Durch die Besiedlung der dreidimensionalen Matrix mit körpereigenen Stammzellen (Knochenersatz). die sich - angeregt durch die jeweiligen Wachstumsfaktoren des umliegenden Gewebes - erst am Ort des Transplantats differenzieren oder durch Besiedelung mit Knorpelzellen zur erneuten Bildung hyalinen Knorpels sind Abstoßungsreaktionen des Transplantats weitgehend ausgeschlossen. Dies ist ein großer Vorteil gegenüber bisherigen Matrices aus verschiedenen Kunststoffen oder Keramiken. Diese Materialien verbleiben im Körper und können noch nach Jahren als fremd erkannt und abgestoßen werden. Die dreidimensionale Matrix kann sowohl mit humanen als auch mit animalischen Zellen (beispielsweise vom Pferd, Hund oder Hai) besiedelt werden. Besonders geeignet sind Haizellen, da diese beim Empfänger keine wesentliche immunologische Antwort auslösen. Haizellen werden bereits als Organersatz verwendet, z. B. für Augenlinsen.

Ein erfindungsgemäßes Implantat, das nur eine geringe bis überhaupt keine Immunantwort stimuliert, ist vorteilhaft aus der oben beschriebenen dreidimensionalen Matrix aufgebaut, die vorzugsweise mit körpereigenen Zellen besiedelt ist. So kann beispielsweise eine dünne, flächige Matrix, die in einem geeigneten Kulturmedium steril mit Keratocyten besiedelt wurde. als Hauttransplantat dienen. Die besiedelte Matrix kann im Operationssaal steril aus dem Kulturmedium entnommen werden und auf die gereinigte Wundfläche aufgebracht werden. In die Matrix können dann körpereigene Zellen unter Ausbildung von Gewebe einwandern.

Möglich ist auch eine Ausführungsform als künstliche Leber. Die Matrix wird dabei in einem geeigneten Kulturmedium mit Leberzellen besiedelt. Der Zellverbund, der noch durch die Matrix stabilisiert sein kann, wird dann in den Körper eines Patienten eingepflanzt und kann dort beispielsweise eine geschädigte Leber in ihrer Funktion unterstützen.

Erfolge wurden erreicht, wenn die dreidimensionale Matrix mit autologen Chondrozyten besiedelt ist. Die Matrix kann dann an den entsprechenden Stellen auf den Knorpel transplantiert werden, wo sich dann neue Knorpelsubstanz entwickeln kann. Dies bietet große Vorteile gegenüber der derzeit üblichen Methode der Transplantation von Knorpelzellen ohne Trägermatrix. Die bei diesem Verfahren bestehende hohe Wahrscheinlichkeit einer Verletzung der Knochenhaut und nachfolgendem Einwachsen von Knochenzellen in die neue Knorpelschicht mit folgender Degenerierung des neuen Knorpels wird durch das erfindungsgemäße Implantat vermieden.

Beispiele für weitere Zellen, die geeignet für eine Besiedlung der Matrix sind, sind Osteocyten, Keratinocyten, Hepatozyten, Knochenmarksstammzellen oder neuronale Zellen.

Die Erfindung wird im Weiteren unter Bezugnahme auf mehrere Figuren und anhand von Beispielen genauer erläutert. Die Figuren zeigen im Einzelnen:
**Fig. 1** eine Abbildung einer Matrix mit Lactat als Gegenion, wobei bei der Herstellung der Matrix die wässrige Chitosanlösung vor dem Einfrieren neutralisiert wurde;
**Fig. 2** eine Abbildung einer Matrix, wobei bei der Herstellung der Matrix die Matrix nach dem Lyophilisieren mit NaOH (5 Gew.-%) äquilibriert wurde: das Gegenion wird bei (a) durch Lactat und bei (b) durch Phosphat gebildet;
**Fig. 3** eine mikrospkopische Aufnahme einer mit Chondrozyten besiedelten Matrix.

### Beispiel 1: Allgemeine Herstellvorschrift zur Herstellung einer dreidimensionalen Matrix (1. Ausführungsform)

Chitosan wird in verdünnter wässriger Säure bei einem pH zwischen 3 und 6 unter Rühren gelöst. Anschließend wird mit verdünnter NaOH der gewünschte pH-Wert eingestellt. Die Lösung wird in ein geeignet geformtes Gefäß überführt und bei -30 bis -15°C eingefroren. Das Gefäß mit der gefrorenen Lösung wird in einen Lyophilisator überführt und das Wasser unter vermindertem Druck (0,01 bis 0.5 hPa) absublimiert. Der schwammartige Rückstand kann ohne weitere Reinigung für die Besiedlung mit Zellen eingesetzt werden.

### Beispiel 2: Allgemeine Herstellvorschrift zur Herstellung einer dreidimensionalen Matrix (2. Ausführungsform)

Chitosan wird in verdünnter wässriger Hydroxycarbonsäure bei einem pH zwischen 3 und 6 unter Rühren gelöst. Die klare Lösung wird bei einer Temperatur von -30 bis -15°C in einem geeignet geformten Gefäß eingefroren. Das Gefäß mit der gefrorene Lösung wird in einen Lyophilisator überführt und das Wasser unter vermindertem Druck (0,01 bis 0.5 hPa) absublimiert. Der nach dem Absublimieren des Wassers zurückbleibende schwammartige Rückstand wird für 24 Stunden in Natronlauge (5 Gew.-%) alkalisiert. Anschließend wird die schwammartige Matrix mit entionisiertem Wasser gewaschen bis auf einen für Zellen verträglichen pH-Wert.

### Beispiel 3: Herstellung einer porösen Chitosanmatrix (Gegenion: Lactat)

0,1 g Chitosan und 0.1113 g Milchsäure (90 % in Wasser, bezogen von Merck KGaA, Darmstadt) wurden in 9.79 g entionisiertem Wasser unter Rühren gelöst und der pH-Wert mit 1 M Natronlauge auf 7 eingestellt. Die klare Lösung wurde zunächst bei -24 °C eingefroren und anschließend bei einem Druck von 0,05 hPa das Lösungsmittel absublimiert.

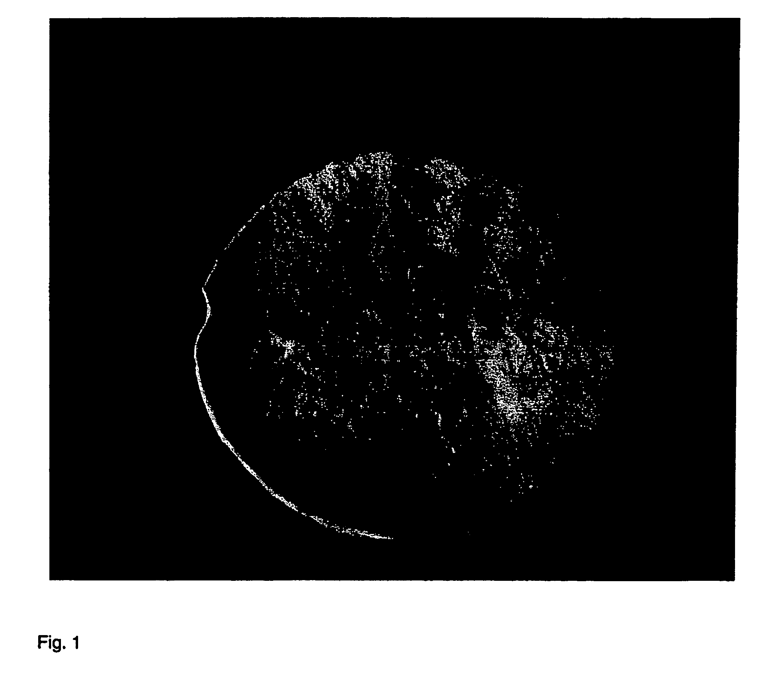

Erhalten wurde ein weißer feinporiger Schaum, der eine schwammartige Elastizität aufwies. Der Schaum wurde ohne weitere Reinigung für die Besiedelung mit Zellen verwendet. Die Matrix ist in Fig. 1 abgebildet. Sie weist eine gleichmäßige, fein stukturierte Oberfläche auf. Es sind keine Verwerfungen festzustellen, die Matrix zeigt durchgehend eine gleichmäßige feinporige Struktur.

### Beispiel 4: Herstellung einer poröser Chitosanmatrix (Gegenion: Acetat) (1. Ausführungsform)

0,1 g Chitosan und 0,1 g Eisessig (100 %ig) wurden in 9,79 g entionisiertem Wasser unter Rühren gelöst und der pH-Wert mit 1 M Natronlauge auf 7 eingestellt. Die klare Lösung wurde zunächst bei -24 °C eingefroren und anschließend bei einem Druck von 0,05 hPa das Lösungsmittel absublimiert.
Erhalten wurde ein weißer feinporiger Schaum.

### Beispiel 5: Herstellung einer porösen Chitosanmatrix (Gegenion: Phosphat)

0,1 g Chitosan und 0,037 ml ortho-Phosphorsäure (85 % Sigma P 6560) wurden in 7,00 g entionisiertem Wasser unter Rühren gelöst und der pH-Wert mit 1 M Natronlauge auf 7 eingestellt. Die klare Lösung wurde zunächst bei -24 °C eingefroren und anschließend bei einem Druck von 0,05 hPa das Lösungsmittel absublimiert.

Erhalten wurde ein weißer feinporiger Schaum, der eine schwammartige Elastizität aufwies. Der Schaum wurde ohne weitere Reinigung für die Besiedelung mit Zellen verwendet.

### Beispiel 6: Herstellung einer porösen Chitosanmatrix (Gegenion: Glutamat)

0,1 g Chitosan und 0,1 g Glutaminsäure (Sigma G 6904) wurden in 10,00 g entionisiertem Wasser unter Rühren gelöst und der pH-Wert mit 1 M Natronlauge auf 7 eingestellt. Die klare Lösung wurde zunächst bei -24 °C eingefroren und anschließend bei einem Druck von 0,05 hPa das Lösungsmittel absublimiert.

Erhalten wurde ein weißer feinporiger Schaum, der eine schwammartige Elastizität aufwies.

### Beispiel 7:

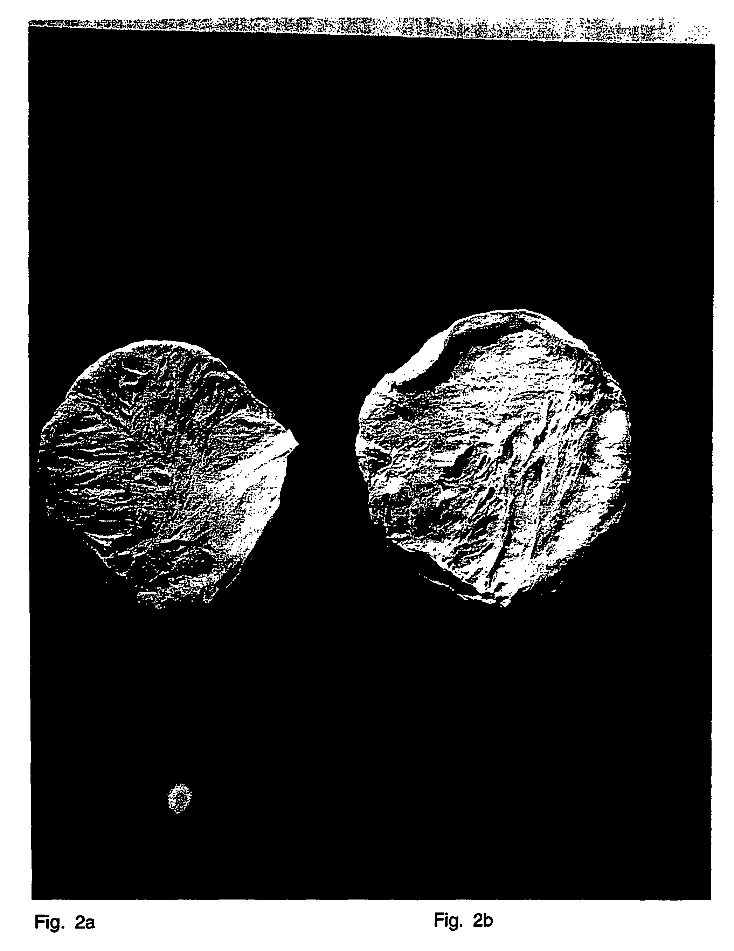

0,1 g Chitosan und 0.1113 g Milchsäure wurden in 9,79 g entionisiertem Wasser unter Rühren gelöst und die klare Lösung zunächst bei -24 °C eingefroren. Anschließend wurde bei einem Druck von 0.05 hPa das Lösungsmittel absublimiert. Der schwammartige Rückstand wurde für 24 Stunden in Natronlauge (5 Gew.-%) alkalisiert und anschließend mit entionisiertem Wasser neutral gewaschen. Nach dem Trocknen wurde ein elastischer weisser Schaum erhalten. Die Matrix ist in Fig. 2a abgebildet.

### Vergleichsbeispiel 1:

0,1 g Chitosan und 0,037 ml ortho-Phosphorsäure wurden in 7,00 g entionisiertem Wasser unter Rühren gelöst und die klare Lösung zunächst bei -24 °C eingefroren. Anschließend wurde bei einem Druck von 0,05 hPa das Lösungsmittel absublimiert. Der schwammartige Rückstand wurde für 24 Stunden in Natronlauge alkalisiert und anschließend mit entionisiertem Wasser neutral gewaschen. Nach dem Trocknen wurde ein brüchiger weisser Schaum erhalten. Die Matrix ist in Fig. 2b abgebildet.

### Beipiel 8: Herstellung einer porösen Chitosanmatrix mit anionischem Charakter (Immobilisierung von L-Glutaminsäure)

0,1 g Chitosan wurden in 10 ml Wasser gegeben und der pH mit 1 M Salzsäure auf 4 eingestellt und die Mischung wurde für 24 Stunden gerührt. Durch Zugabe von 1 N NaOH wurde der pH auf 5,8 eingestellt und dann unter Rühren 0,05 g Glutaminsäure und 0,05 g 1-Ethyl-3-(3-Dimethylaminopropyl) Carbodiimid (EDAC) zugegeben und gelöst. Nachdem für weitere 3 Stunden bei Raumtemperatur gerührt worden war, wurde, der pH mit 1 M NaOH auf 7 eingestellt, der Niederschlag abfiltriert und mit 2 l entionisiertem Wasser gewaschen. Der gewaschene Niederschlag wurde mit 0,1113 g Milchsäure (90 % in Wasser, bezogen von Merck KGaA, Darmstadt) in 9.79 g Wasser unter Rühren gelöst. Der pH wurde mit 1 N Natronlauge auf 7 eingestellt und die Lösung bei -24 °C eingefroren. Anschließend wurde das Wasser bei einem Druck von 0,05 hPa absublimiert.

Es wurde ein weißer schwammartiger Rückstand erhalten. Der Schwamm kann ohne weitere Reinigung verwendet werden.

### Beispiel 9: Herstellung einer dreidimensionalen Matrix mit immobilisierten Zelladhäsionsfaktoren L-Arg-L-Gly-L-Asp

0,1 g Chitosan wurden in 10 ml Wasser gegeben und der pH mit 1 M Salzsäure auf 4 eingestellt. Nachdem 24 Stunden bei Raumtemperatur gerührt wurde, wurde der pH der klaren Lösung mit 1 M NaOH auf 5,8 eingestellt und anschließend nacheinander 0,01 g L-Arg-L-Gly-L-Asp und 0.05 g 1-Ethyl-3-(3-Dimethylaminopropyl) Carbodiimid (EDAC) zugegeben und unter Rühren gelöst. Nachdem für weitere 3 Stunden bei Raumtemperatur gerührt worden war, wurde der pH mit 1 N NaOH auf 7 eingestellt. Der Niederschlag wurde abfiltriert und mit 2 l Wasser gewaschen. Anschließend wurde der Niederschlag mit 0,1113 g Milchsäure (90 % in Wasser, bezogen von Merck KGaA, Darmstadt) in 9,79 g Wasser unter Rühren gelöst, der pH mit 1 N Natronlauge auf 7 eingestellt und die Lösung bei -24 °C eingefroren. Schließlich wurde das Wasser bei einem Druck von 0,05 hPa absublimiert.

Es wurde ein weißer schwammartiger Rückstand erhalten. Der Schwamm kann ohne weitere Reinigung verwendet werden.

### Beispiel 10: Allgemeine Arbeitsvorschrift für die Aussaat und Inkubation von Zellen auf der dreidimensionalen Matrix.

Eine gemäß den Beispielen 1 bis 6 erhaltene dreidimensionale Matrix wird in PBS bei 121 °C für 20 Minuten autoklaviert. Die Matrix ist anschließend steril und vollständig mit Flüssigkeit benetzt. Anschließend werden Zellen, die in einem geeigneten Kulturmedium kultiviert wurden, direkt auf eine den Bedürfnissen entsprechend zugeschnittene Matrix gegeben und bei der geeigneten Temperatur inkubiert. Die bewachsene Matrix kann steril entnommen und beispielsweise implantiert werden.

### Beispiel 11: Kultivierung von Chondrozyten auf der dreidimensionalen Matrix.

Autologe Chondrozyten, die durch Biopsie gewonnen wurden, wurden auf Chondrozyte Growth Medium (CellApplications Inc., USA) kultiviert.

Eine nach Beispiel 3 hergestellte dreidimensionale Matrix wurde zunächst bei 121 °C für 20 Minuten autoklaviert. Die vollständig benetzte Matrix wurde unter sterilen Bedingungen entsprechend zugeschnitten und in ein Kulturgefäß gegeben. Anschließend wurden die kultivierten Chondrozyten mit dem Kulturmedium auf die dreidimensionale Matrix gegeben und für 14 bis 21 Tage bei 37 °C inkubiert. Innerhalb dieses Zeitraums wuchsen die Zellen in die dreidimensionale Matrix ein. Die bewachsene Matrix wurde steril entnommen und kann anschließend implantiert werden.

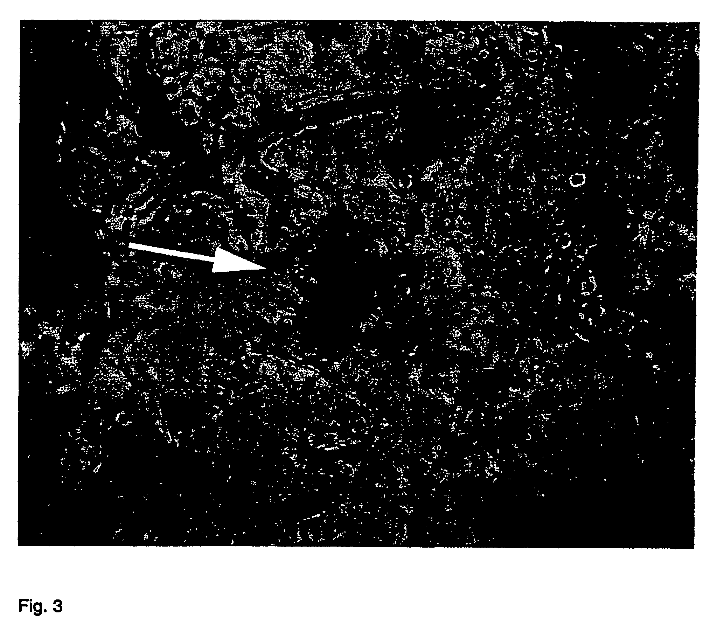

In Fig. 3 ist die besiedelte Chitosanmatrix als mikroskopisch vergrößerte Aufnahme gezeigt. Die durch einen Pfeil gekennzeichneten dunkleren Bereiche aus kugelförmigen Aggregaten sind Zellaggregate, die sich während der Kultivierung auf der Matrix entwickelt haben. Die Zellen wachsen in die Poren der Matrix hinein und bilden dort dreidimensionale Zellaggregate aus.

Die Eigenschaften der Chitosanmatrix werden stark vom Herstellverfahren sowie vom verwendeten Gegenion beeinflusst. Der Einfluss des Gegenions wird aus einem Vergleich der Fig. 2a (Beispiel 7) und 2b (Vergleichsbeispiel 1) deutlich. Beide Matrices wurden hergestellt, indem Chitosan und die entsprechende Säure in Wasser gelöst wurden, die Lösung eingefroren und das Lösungsmittel absublimiert wurde. Anschließend wurden die schaumartigen Rückstände in wässriger NaOH äquilibriert. Bei Verwendung von Lactat als Gegenion (Fig. 2a) fällt die Strukturveränderung der Matrix beim Äquilibrieren mit NaOH deutlich geringer aus als bei Verwendung von Phosphat (Fig. 2b). Die Oberfläche der Matrix ist glatter und zeigt eine geringer ausgeprägte Strukturierung. Die in Beispiel 7 erhaltene Matrix ist deutlich elastischer und stabiler als die Matrix aus dem Vergleichsbeispiel 1. Sie lässt sich zusammendrücken und kehrt bei nachlassendem Druck wieder elastisch in die ursprüngliche Form zurück. Ebenso lässt sie sich elastisch verbiegen, ohne dabei zu zerbrechen.

Der Einfluss des Herstellungsverfahrens wird durch Vergleich der Fig. 1 und 2a deutlich. Bei Fig. 1 (Beispiel 3) erfolgt die Neutralisierung der Milchsäure vor dem Lyophilisieren, während bei der Matrix aus Fig. 2a die Milchsäure nach dem Lyopholisieren durch Alkalisieren mit wässriger NaOH entfernt wurde. In Fig. 1 ist deutlich die gleichmäßige Struktur der Oberfläche der Matrix zu sehen, die nahezu frei von größeren Vertiefungen ist. Die in Fig. 1 dargestellte Matrix zeigt gegenüber der Matrix aus Fig. 2a eine weiter verbesserte Weichheit und Elastizität.

Die Eigenschaften der Matrices sind in einem qualitativen Vergleich in Tabelle 1 zusammengefasst.

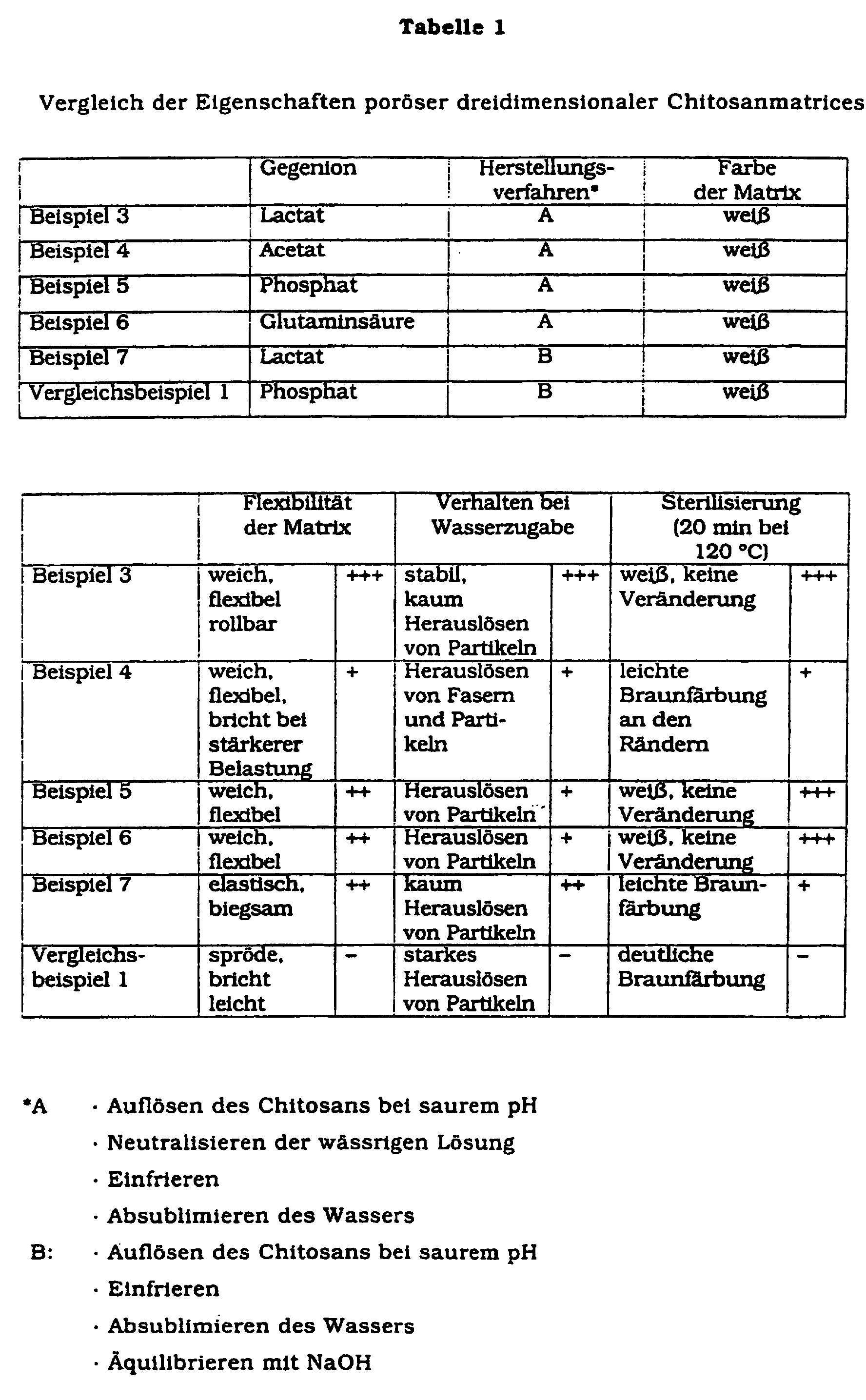

## Patentansprüche

1. Verfahren zur Herstellung einer biologisch verträglichen dreidimensionalen Matrix, wobei
aus Chitosan und einem Überschuss einer Säure eine wässrige Lösung hergestellt wird, die wässrige Lösung neutralisiert wird,
die neutralisierte wässrige Lösung eingefroren wird und
das Wasser unter vermindertem Druck absublimiert wird.

2. Verfahren zur Herstellung einer biologisch verträglichen dreidimensionalen Matrix, wobei
aus Chitosan und einem Überschuss einer Säure, die ausgewählt ist aus der Gruppe, die gebildet ist von Alkyl- und Arylhydroxycarbonsäuren, eine wässrige Lösung hergestellt wird,
die wässrige Lösung eingefroren wird und
das Wasser unter vermindertem Druck absublimiert wird, wobei
überschüssige Säure vor oder nach dem Absublimieren entfernt wird.

3. Verfahren nach Anspruch 1 oder 2, wobei die Säure Milchsäure ist.

4. Verfahren nach einem der vorhergehenden Ansprüche, wobei zur Entfernung oder Neutralisation der Säure der pH-Wert der wässrigen Lösung durch Zugabe einer Base auf 5,0 bis 7,5, vorzugsweise 5, 5 bis 7,0, insbesondere 6,0 bis 7,0 eingestellt wird.

5. Dreidimensionale Matrix, erhältlich durch ein Verfahren gemäß einem der Ansprüche 1 bis 4.

6. Dreidimensionale Matrix nach Anspruch 5, wobei Adhäsionsfaktoren, Hormone und/oder Wachstumsfaktoren an das Chitosangerüst gebunden sind.

7. Dreidimensionale Matrix nach Anspruch 5, wobei die Matrix mit autologem Fibrin beschichtet ist.

8. Verwendung der dreidimensionalen Matrix gemäß einem der Ansprüche 5 bis 7 als Festphase in einem Kulturreaktor.

9. Verwendung der dreidimensionalen Matrix gemäß einem der Ansprüche 5 bis 7 als Matrix für Implantate.

10. Implantat, umfassend eine dreidimensionale Matrix gemäß einem der Ansprüche 5 bis 7.

## Claims

1. A method for producing a biocompatible three-dimensional matrix, where
an aqueous solution is prepared from chitosan and an excess of an acid, the aqueous solution is neutralized, the neutralized aqueous solution is frozen, and the water is removed by sublimation under reduced pressure.

2. A method for producing a biocompatible three-dimensional matrix, where
an aqueous solution is prepared from chitosan and an excess of an acid which is selected from the group formed by alkyl and aryl hydroxy carboxylic acids,
the aqueous solution is frozen and the water is removed by sublimation under reduced pressure, with excess acid being removed before or after the removal by sublimation.

3. A method as claimed in claim 1 or 2, where the acid is lactic acid.

4. A method as claimed in any of the preceding claims, where the acid is removed or neutralized by adjusting the pH of the aqueous solution by adding a base to from 5.0 to 7.5, preferably 5.5 to 7.0, in particular 6.0 to 7.0.

5. A three-dimensional matrix obtainable by a method as claimed in any of claims 1 to 4.

6. A three-dimensional matrix as claimed in claim 5, where adhesion factors, hormones and/or growth factors are bound to the chitosan framework.

7. A three-dimensional matrix as claimed in claim 5, where the matrix is coated with autologous fibrin.

8. The use of the three-dimensional matrix as claimed in any of claims 5 to 7 as solid phase in a culture reactor.

9. The use of the three-dimensional matrix as claimed in any of claims 5 to 7 as matrix for implants.

10. An implant comprising a three-dimensional matrix as claimed in any of claims 5 to 7.

## Revendications

1. Procédé de préparation d'une matrice tridimensionnelle biologiquement compatible, dans lequel on prépare à partir de chitosane et d'un excès d'un acide une solution aqueuse, on neutralise la solution aqueuse, on congèle la solution aqueuse neutralisée, et on chasse l'eau par sublimation sous pression réduite.

2. Procédé de préparation d'une matrice tridimensionnelle biologiquement compatible, dans lequel on prépare à partir de chitosane et d'un excès d'un acide qui est choisi dans l'ensemble constitué par les acides alkyl- et arylhydroxycarboxylique, une solution aqueuse, on congèle la solution aqueuse, et on chasse l'eau par sublimation sous pression réduite, l'acide en excès étant éliminé avant ou après sublimation.

3. Procédé selon la revendication 1 ou 2, dans lequel l'acide est l'acide lactique.

4. Procédé selon l'une des revendications précédentes, dans lequel, pour éliminer ou neutraliser l'acide, on ajuste le pH de la solution aqueuse, par addition d'une base, à une valeur de 5,0 à 7,5, de préférence de 5,5 à 7,0, en particulier de 6,0 à 7,0.

5. Matrice tridimensionnelle pouvant être obtenue par un procédé selon l'une des revendications 1 à 4.

6. Matrice tridimensionnelle selon la revendication 5, dans laquelle des facteurs d'adhérence, des hormones et/ou des facteurs de croissance sont liés au squelette du chitosane.

7. Matrice tridimensionnelle selon la revendication 5, dans laquelle la matrice est revêtue de fibrine autologue.

8. Utilisation de la matrice tridimensionnelle selon l'une des revendications 5 à 7 en tant que phase solide dans un réacteur de culture.

9. Utilisation de la matrice tridimensionnelle selon l'une des revendications 5 à 7 en tant que matrice pour implants.

10. Implant comprenant une matrice tridimensionnelle selon l'une des revendications 5 à 7.
